# EUROPÄISCHE PATENTANMELDUNG

(11) **EP 1 081 139 A2**
(43) Veröffentlichungstag der Anmeldung: **07.03.2001**
(21) Anmeldenummer: 00117912.6
(22) Anmeldetag: 21.08.2000
(51) Int. Cl.: C07D 233/32

(54) **Herstellung einer Imidazolidindicarbonsaüre**

(30) Priorität: 27.08.1999 EP 99116967
(71) Anmelder: F. HOFFMANN-LA ROCHE AG, 4070 Basel (CH)
(72) Erfinder: Behringer, Klaus, 4052 Basel (CH)

(57) **Zusammenfassung**

Ein Verfahren zur Herstellung von 2-Oxo-1, 3-dibenzyl-cis-4,5-imidazolidindicarbonsäure ausgehend von meso-2,3-Bis(benzylamino)-bernsteinsäure-Dialkalimetallsalz besteht darin, dass man man das meso-2,3-Bis(benzylamino)-bernsteinsäure-Dialkalimetallsalz mit Triphosgen in einem aus wässriger Alkalilauge und einem organischen Lösungsmittel bestehenden Zweiphasen-Lösungsmittelsystem bei einer etwa 50°C nicht übersteigenden Temperatur umsetzt und das daraus resultierende, in der wässrigen Phase befindliche 2-Oxo-1,3-dibenzyl-cis-4,5-imidazolidindicarbonsäure-Dialkalimetallsalz durch Ansäuern in die gewünschte 2-Oxo-1,3-dibenzyl-cis-4,5-imidazolidindicarbonsäure umwandelt. Das Produkt ist ein wichtiges Zwischenprodukt im mehrstufigen Verfahren zur Herstellung von Biotin (Vitamin H).

## Beschreibung

Die vorliegende Erfindung betrifft ein Verfahren zur Herstellung einer Cyclosäure, nämlich von 2-Oxo-1,3-dibenzyl-cis-4,5-imidazolidindicarbonsäure, die ein wichtiges Zwischenprodukt im mehrstufigen Verfahren zur Herstellung von Biotin (Vitamin H) darstellt.

Die Herstellung der oben erwähnten Cyclosäure ausgehend von meso-2,3-Bis(benzylamino)-bernsteinsäure in Form ihres Dialkalimetallsalzes ist bekannt. So beschreibt beispielsweise die US-Patentschrift Nr. 5.151.525 ein derartiges Verfahren, in welchem Phosgen als Reagens zur Verknüpfung der beiden sekundären Stickstoffatome über eine Carbonylgruppe, und daher zur Ringbildung, in einem alkalischen wässrig/organischen Zweiphasen-Lösungsmittelsystem verwendet wird. Dabei wird als im wesentlichen nicht-wassermischbares Lösungsmittel für die Umsetzung Anisol eingesetzt.

Bekanntlich ist das Reagens Phosgen jedoch hochtoxisch und zudem unter Einwirkung anderer Gase oder gewisser Reaktionsflüssigkeiten potentiell explosiv, so dass dessen Verwendung bei unsorgfältiger Handhabung bzw. Ueberwachung äusserst gefährlich ist, und besondere Massnahmen bei dessen Transport, Lagerung und Verwendung, z.B. apparative Sicherheitsvorrichtungen, erforderlich sind.

Der vorliegenden Erfindung liegt nun die Aufgabe zugrunde, ein Verfahren zur Herstellung von 2-Oxo-1,3-dibenzyl-cis-4,5-imidazolidindicarbonsäure ausgehend von meso-2,3-Bis(benzylamino)-bernsteinsäure in Form ihres Dialkalimetallsalzes und unter Verwendung eines alternativen Reagens zur oben näher erläuterten Ringbildung, wobei das Reagens die Nachteile der Verwendung von Phosgen nicht oder zumindest viel weniger aufweist. Diese Aufgabe wird überraschend gut gelöst, indem man als alternatives Reagens Kohlensäure-bis(trichlormethylester), auch als "Bis(trichlormethyl)carbonat" oder - ganz knapp und im folgenden mehrmals erwähnt - "Triphosgen" bekannt, verwendet und ansonsten das Verfahren unter gewissen Reaktionsbedingungen durchführt.

Bei dem erfindungsgemässen Verfahren handelt es sich um ein Verfahren zur Herstellung von 2-Oxo-1,3-dibenzyl-cis-4,5-imidazolidindicarbonsäure ausgehend von meso-2,3-Bis(benzylamino)-bernsteinsäure-Dialkalimetallsalz, das dadurch gekennzeichnet ist, dass man das meso-2,3-Bis(benzylamino)-bernsteinsäure-Dialkalimetallsalz mit Triphosgen in einem aus wässriger Alkalilauge und einem organischen Lösungsmittel bestehenden Zweiphasen-Lösungsmittelsystem bei einer etwa 50°C nicht übersteigenden Temperatur umsetzt und das daraus resultierende, in der wässrigen Phase befindliche 2-Oxo-1,3-dibenzyl-cis-4,5-imidazolidindicarbonsäure-Dialkalimetallsalz durch Ansäuern in die gewünschte 2-Oxo-1,3-dibenzyl-cis-4,5-imidazolidindicarbonsäure umwandelt.

Das nachfolgende Reaktionsschema stellt das erfindungsgemässe Verfahren strukturell dar:

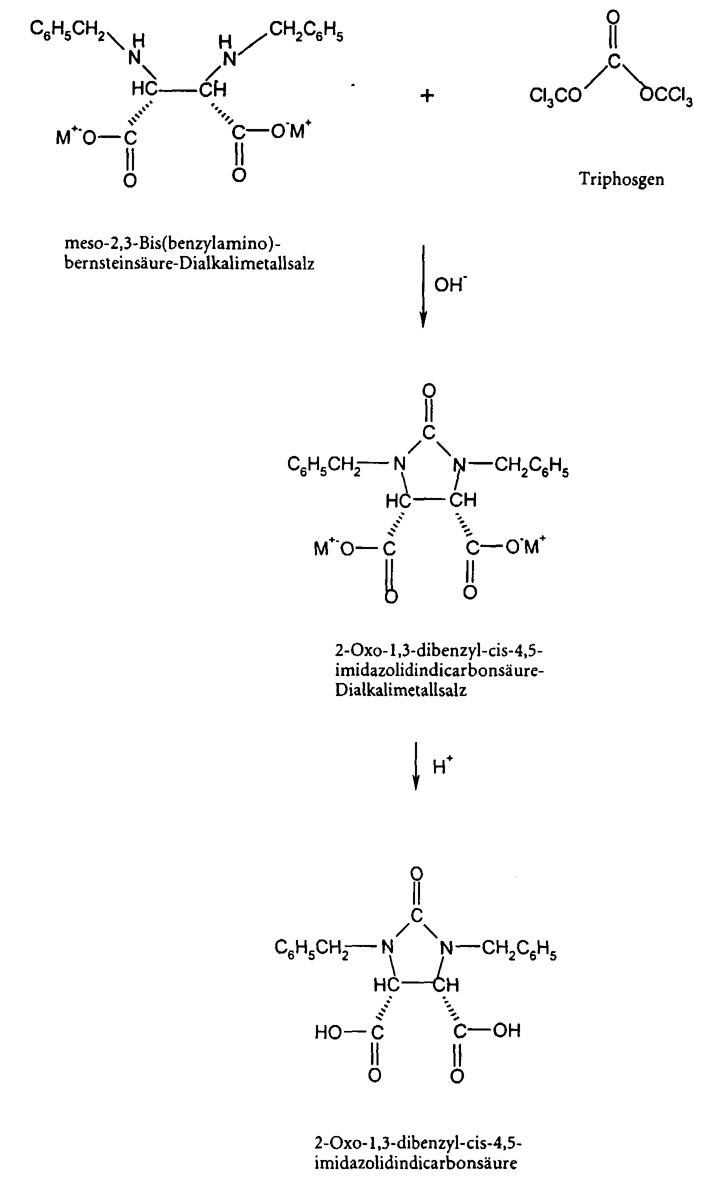

Im obigen Reaktionsschema ist das Alkalimetallion M⁺ zweckmässigerweise entweder das Natrium- oder das Kaliumion, vorzugsweise das Kaliumion, so dass als Ausgangsmaterial des erfindungsgemässen Verfahrens zweckmässigerweise das Dinatrium- oder Dikaliumsalz, vorzugsweise das letztere, verwendet wird.

Das als Reagens im erfindungsgemässen Verfahren verwendete Triphosgen ist in gereinigtem Zustand ein weisses, kristallines Produkt mit einem Schmelzpunkt von 78 - 80°C. Es lässt sich unzersetzt destillieren, und zwar beim Siedepunkt 203 - 206°C unter atmosphärem Druck (760 mm Hg / 0,1013 MPa). Dessen Zersetzungstemperatur beträgt über 350°C. Triphosgen zersetzt sich nur langsam an der Luft und lässt sich damit an der Luft bedeutend besser handhaben als Phosgen. Es lässt sich bekanntlich durch Photochlorierung von Dimethylcarbonat in guter Ausbeute und Qualität herstellen, und ist seit Jahren auch in grösseren Mengen im Handel erhältlich.

Zur Zubereitung des meso-2,3-Bis(benzylamino)-bernsteinsäure-Dialkalimetallsalzes wird zweckmässigerweise die Säure selber in Wasser, vorzugsweise entionisiertem Wasser, aufgeschlämmt und die resultierende Aufschlämmung mit Alkalimetalllauge, d.h. zweckmässigerweise Natron- oder Kalilauge, vorzugsweise Kalilauge, versetzt, und zwar im allgemeinen bei einem pH-Wert von etwa 9 bis etwa 14, vorzugsweise bei einem pH-Wert von etwa 12 bis etwa 13. Dies ergibt eine klare, alkalische wässrige Lösung des erwünschten Dialkalimetallsalzes. Dabei werden geeignete Mengen an Wasser und zugegebener Alkalimetalllauge verwendet, um zweckmässigerweise zu erreichen, dass die Konzentration des gebildeten meso-2,3-Bis(benzylamino)-bernsteinsäure-Dialkalimetallsalzes etwa 5 bis etwa 20 Gewichtsprozent, vorzugsweise etwa 10 bis etwa 15 Gewichtsprozent, bezogen auf das Gesamtgewicht der entstehenden klaren alkalischen wässrigen Lösung bei pH etwa 9 bis etwa 14, beträgt. Die Konzentration der zugegebenen Alkalimetalllauge ist nicht kritisch, beträgt allerdings etwa 45 - 50 Gewichtsprozent im Falle der Verwendung der handelsüblichen Alkalimetalllauge, z.B. Kalilauge.

Das aus wässriger Alkalilauge und einem organischen Lösungsmittel bestehende Zweiphasen-Lösungsmittelsystem, in welchem das meso-2,3-Bis(benzylamino)-bernsteinsäure-Dialkalimetallsalz und das Triphosgen in erfindungsgemässen Verfahren miteinander reagieren, wird zweckmässigerweise durch Zusammenbringen der oben beschriebenen klaren alkalischen wässrigen Lösung des ersteren Reaktionsteilnehmers mit einer Lösung des Triphosgens im organischen Lösungsmittel gebildet. Als organisches Lösungsmittel wird zweckmässigerweise ein aprotisches organisches Lösungsmittel, insbesondere ein aliphatischer oder cyclischer Ether, z.B. Diethylether bzw. Tetrahydrofuran oder Dioxan; ein aliphatischer oder alicyclischer Kohlenwasserstoff, z.B. Hexan, Octan bzw. Cyclohexan; ein aliphatischer oder cyclischer Ester, z.B. Ethylacetat bzw. γ-Butyrolacton; oder ein aromatischer Kohlenwasserstoff, z.B. Benzen oder Toluen, verwendet. Es wird als organisches Lösungsmittel vorzugsweise Tetrahydrofuran oder Toluen verwendet. Die Konzentration der Lösung des Triphosgens im Lösungsmittel kann in einem breiten Bereich variieren, dessen oberer Grenzwert natürlich vom jeweiligen Lösungsmittel abhängt. Diese Konzentration ist zwar nicht kritisch, ist allerdings aus ökologischen und wirtschaftlichen Gründen eher so hoch wie möglich.

Bei dem Zusammenbringen der alkalischen wässrigen Lösung des meso-2,3-Bis(benzylamino)-bernsteinsäure-Dialkalimetallsalzes mit der Lösung des Triphosgens im organischen Lösungsmittel ist zweckmässigerweise die erstere Lösung bereits auf eine erhöhte Temperatur im Bereich von etwa 30 bis etwa 50°C, vorzugsweise im Temperaturbereich von etwa 40 bis etwa 45°C, erwärmt worden. Auch die Lösung des Triphosgens kann gewünschtenfalls im voraus auf die entsprechende Temperatur erwärmt werden. Als weitere Möglichkeit kann das Erwärmen erst im Laufe des Zusammenbringens oder danach erfolgen.

Das Zusammenbringen kann in beliebiger Reihenfolge geschehen, d.h. man kann die Lösung des Triphosgens zu der alkalischen wässrigen Lösung des meso-2,3-Bis(benzylamino)-bernsteinsäure-Dialkalimetallsalzes, oder die alkalische wässrige Lösung zu der Lösung des Triphosgens geben. Vorzugsweise wird die erstere Zugabevariante vorgenommen. Dabei erweist es sich als vorteilhaft, die Lösung des Triphosgens eher langsam und kontinuierlich, z.B. tropfenweise, zuzugeben. Um ein gutes Durchmischen während des Zusammenbringens zu erreichen, wird geeigneterweise gerührt oder anderweitig durchmischt.

Was die relativen Mengen an meso-2,3-Bis (benzylamino)-bernsteinsäure-Dialkalimetallsalz und Triphosgen nach vollendetem Zusammenbringen anbelangt, so beträgt das Molverhältnis Triphosgen : meso-2,3-Bis (benzylamino)-bernsteinsäure-Dialkalimetallsalz geeigneterweise etwa 0,33 : 1 bis etwa 10 : 1, vorzugsweise etwa 1,5 : 1 bis etwa 5 : 1. Der Bereich von etwa 2 : 1 bis etwa 4 : 1 ist besonders bevorzugt.

Während der Umsetzung des Triphosgens mit dem meso-2,3-Bis(benzylamino)-bernsteinsäure-Dialkalimetallsalz im Zweiphasen-Lösungsmittelsystem wird der pH-Wert der wässrigen Phase zweckmässigerweise im Bereich von etwa 8,5 bis etwa 13, vorzugsweise im Bereich von etwa 9,5 bis etwa 10, 5, gehalten. Zur Beibehaltung dieses pH-Bereiches wird zweckmässigerweise wässrige Natrium- oder Kaliumhydroxidlösung nötigenfalls zugegeben, und zwar gleichzeitig mit dem Zusammenbringen der Reaktionsteilnehmer oder nach Beendigung des Zusammenbringens. Die Konzentration der Natrium- oder Kaliumhydroxidlösung ist nicht kritisch, beträgt allerdings geeigneterweise etwa 5 bis etwa 50 Gewichtsprozent. Besonders geeignet wird dazu die gleiche Natron- bzw. Kalilauge verwendet wie diejenige, welche zur Zubereitung des meso-2,3-Bis (benzylamino)-bernsteinsäure-Dialkalimetallsalzes verwendet wurde.

Die Umsetzung erfolgt bei einer etwa 50°C nicht übersteigenden Temperatur, und zwar im allgemeinen bei Temperaturen im Bereich von etwa 30 bis etwa 50°C, vorzugsweise bei Temperaturen von etwa 40 bis etwa 45°C. Der Druck ist nicht kritisch; es wird normalerweise unter atmosphärem oder leicht erhöhtem Druck verfahren.

Die Durchführung des erfindungsgemässen Verfahrens kann gewünschtenfalls unter einer Inertgasatmosphäre erfolgen. Im Falle der Verwendung einer Inertgasatmosphäre eignet sich als Inertgas insbesondere Stickstoff oder Argon, wobei im industriellen Massstab Stickstoff bevorzugt ist.

Nach erfolgter Zugabe (Zusammenbringen der beiden Lösungen), welche in der Regel etwa 2 bis 4 Stunden dauert, ist die Reaktion normalerweise auch beendet. Das resultierende Zweiphasen-Gemisch kann dann aufgearbeitet werden. Das gewünschte Produkt 2-Oxo-1,3-dibenzyl-cis-4,5-imidazolidindicarbonsäure in Form ihres Dialkalimetallsalzes befindet sich hauptsächlich in der wässrigen Phase und wird durch Ansäuern dieser Phase ausgefällt, und zwar in Form der freien Säure. Es kann beliebig die vorerst getrennte wässrige Phase oder das ganze Zweiphasen-Gemisch angesäuert werden, wobei im letzteren Fall die entstehende Dicarbonsäure in die organische Phase wandert und daraus isoliert werden muss. Als Säure zum Ansäuern wird zweckmässigerweise eine Mineralsäure verwendet, insbesondere Salzsäure, Bromwasserstoffsäure oder Schwefelsäure, vorzugsweise Salzsäure, deren jeweilige Konzentration und Menge zweckmässigerweise so gewählt werden, dass die wässrige Phase, aus welcher das Produkt ausfällt, schliesslich einen pH-Wert von etwa 0,5 bis etwa 1,0 aufweist; dies fördert ein gutes Ausfallen des Produktes. Auch beim Ansäuern ist die Reihenfolge der Zugabe beliebig.

Falls die wässrige Phase von der organischen Phase vorerst getrennt wird, kann dies auf an sich bekannte Weise erfolgen, z.B. durch Trennung der unteren (schwereren) wässrigen Phase in einem Scheidetrichter, Dekantieren oder Zentrifugation. Andernfalls wird das Produkt nach ebenfalls auf an sich bekannte Weise durchgeführter Abtrennung der angesäuerten wässrigen Phase aus der organischen Phase isoliert; in diesem Fall kann das Produkt beispielsweise vom organischen Lösungsmittel durch Abdestillieren isoliert werden.

Die so isolierte 2-Oxo-1,3-dibenzylamino-cis-4,5-imidazolidindicarbonsäure kann in an sich bekannter Weise gewaschen, zweckmässigerweise mit Wasser, getrocknet und gewünschtenfalls weiter gereinigt werden.

Das erfindungsgemässe Verfahren wird durch die nachfolgenden Beispiele veranschaulicht:

### Beispiel 1

In einem mit Dimrothkühler, 250 ml-Tropftrichter mit Druckausgleich, Thermometer, mechanischem Rührer und pH-Elektrode versehenen 750 ml-Fünfhalssulfierkolben wurden unter Inertgas und Rühren 16,4 g (50 mmol) meso-2,3-Bis(benzylamino)-bernsteinsäure (DBA; etwa 99%ig rein nach HPLC) in 160 ml entionisiertem Wasser aufgeschlämmt. Unter Rühren wurden 12,0 ml 48 Gew.%ige Kalilauge (152,4 mmol KOH) zugefügt, und die resultierende klare Lösung wurde im Oelbad auf 40 - 45°C Innentemperatur erwärmt. Die DBA ging als Dikalium-Salz schwach gelbbraun gefärbt in Lösung; die Lösung wies einen pH-Wert von 13 auf.

In einem 250 ml-Tropftrichter mit Druckausgleich wurden 44,5 g (150 mmol) Triphosgen (98%ig rein nach HPLC) in 120 ml absolutem Tetrahydrofuran (THF) gelöst. Dabei wurde keine Wärmetönung oder Gasentwicklung beobachtet. Man erhielt etwa 150 ml Triphosgen/THF-Lösung.

Man tropfte letztere Lösung innert 2,5 Stunden unter kräftigem Rühren bei einer Innentemperatur von 40 - 45°C zur Lösung des DBA-Dikaliumsalzes zu. Anschliessend wurde mit 48 Gew.%iger Kalilauge mittels Dosimat und pH-Controller bei einem pH-Wert zwischen 9,8 und 10,3 neutralisiert. Mit einem Wasserbad wurde die Temperatur zwischen 40 und 45°C gehalten. Nach Zugabe von etwa 100 ml Triphosgen/THF-Lösung lag das Reaktionsgemisch als schwach braun gefärbte Emulsion vor. Dann wurden zusätzliche 20 ml entionisiertes Wasser zugefügt. Nach etwa 2,5 Stunden war die gesamte Menge Triphosgen/THF-Lösung zugetropft worden.

Zur Neutralisation wurden 140 ml 48 Gew.%ige Kalilauge (1,778 mol KOH) zugegeben. Man liess noch 5 Minuten nachrühren, währenddessen der pH-Wert der Lösung bei 10 blieb.

Zum Ansatz wurden zwecks besserer Trennung der wässrigen Phase von der organischen 70 ml entionisiertes Wasser zugefügt. Nach kurzem Rühren wurde der Ansatz in einen 1000 ml-Scheidetrichter überführt. Man erhielt zwei Phasen: die obere Phase (58 g) enthielt den grössten Teil des THF sowie dunkelbraun gefärbte, wasserunlösliche Verunreinigungen, und nach Einengen unter vermindertem Druck blieb etwa 0,4 g einer festen, braunen Masse zurück. Die untere, wässrige Phase enthielt 2-Oxo-1,3-dibenzyl-cis-4,5-imidazolidindicarbonsäure (CAC) und DBA als Dikalium-Salz gelöst sowie noch gelöstes THF. Dieses THF musste vor der Aufarbeitung abdestilliert werden. Die wässrige Phase wurde aus dem Scheidetrichter wieder in die Apparatur überführt, die Apparatur mit einer absteigenden Destillationbrücke versehen und bei einer Oelbadtemperatur von 85 - 90°C das noch gelöste THF bis zu einer Kopftemperatur von 75°C abdestilliert. Man erhielt etwa 33 g Destillat. Die Menge der verbleibenden Reaktionsiösung betrug 510 g bzw. 470 ml.

Zur Aufarbeitung wurde die Reaktionsiösung bei etwa 40°C unter gutem Rühren innert 2 Stunden mit 65 ml 36%iger Salzsäure (754 mmol HCl) bis auf pH 0,90 angesäuert. Die CAC sowie nicht umgesetzte DBA fielen zuerst als gummiartiger Niederschlag an, der nach 30 minütigem Rühren bei 40°C feinkörnig wurde. Danach wurde noch 30 Minuten im Eisbad gerührt, durch eine P4-Glassinterfritte filtriert und der Frittenrückstand mit 340 ml entionisiertem Wasser neutral gewaschen. Der Rückstand wurde im Trockenschrank unter Hochvakuum und bei 80°C bis zur Gewichtskonstanz getrocknet und danach in 150 ml absolutem Aceton aufgenommen. Es wurde 30 Minuten rückflussiert und dann durch die gleiche Fritte wie vorher verwendet filtriert und dreimal mit je 30 ml absolutem Aceton auf der Fritte gewaschen. Die CAC befand sich in den gelbbraun gefärbten Acetonextrakten; auf der Fritte waren geringe Mengen an DBA zu erkennen. Die Acetonextrakte wurden unter vermindertem Druck bei 45°C Badtemperatur und etwa 110 mbar (11 kPa) Druck eingeengt und danach im Trockenschrank unter Hochvakuum bei 50°C bis zur Gewichtskonstanz getrocknet. Es wurden 17,0 g an roher CAC erhalten (96%ige Ausbeute). Auf der Fritte wurde 0,12 g DBA isoliert (0,7%).

| Ergebnisse | |
|---|---|
| isolierte DBA: | 0,12 g = 0,7% der Theorie |
| rohe CAC: | 17,0 g = 96% der Theorie |
| Gehalt an reiner CAC: | 93,65% (nach HPLC) |
| Ausbeute an reiner CAC: | 90,0% der Theorie |
| Aussehen: | schwach gelbbraunes, amorphes Pulver |
| Geruch: | schwach vorhanden, undefinierbar |
| Braunes aus der THF-Phase isoliertes Nebenprodukt: | 0,4 g = 2,5% bezogen auf DBA; verworfen |
| Menge an wiedergefundem THF: (Die THF-Phase enthielt 57,5 g THF. Die Menge Destillat betrug 33,0 g THF) | 90,5 g = 85% der Theorie |
| Menge Kaliumchlorid im wässrigen Filtrat: (entspricht der Neutralisation von 1,930 mol KOH mit HCl) | 144 g |

Im wässrigen Filtrat sind nach Dünnschichtchromatographie(DC) -Analyse noch maximal 1% DBA sowie 2 - 3% unbekannte Nebenprodukte. Keine CAC war im Filtrat vorhanden. Das Filtrat wurde verworfen.

### Analytik

CAC und DBA lassen sich gut mit der HPLC-Methode bestimmen, und CAC, DBA sowie die braunen Nebenprodukte lassen sich mittels DC qualitativ nachweisen. Unter Verwendung von Merck Kieselgel 60 F₂₅₄ DC-Platten, 50 Toluen / 50 Eisessig / 10 Methanol (jeweils Volumenteile) als Laufmittelgemisch und Aceton als Lösungsmittel für die CAC und die braunen Nebenprodukte bzw. verdünnte Salzsäure als Lösungsmittel für die DBA wurden folgende Rf-Werte ermittelt:

| | |
|---|---|
| DBA | 0,38 |
| CAC | 0,51 |
| Nebenprodukte | 0,84 verschmiert |

Die Laufzeit betrug 45 - 50 Minuten und die Entwicklung erfolgte innert 2 Stunden im Jodtank, was braune Flecken ergab.

Zusammenfassend kann gesagt werden, dass mit 3 Aequivalenten Triphosgen, gelöst in THF, DBA fast vollständig in CAC übergeführt wurde. Der Gehalt an reiner CAC betrug 93,6%, und die Ausbeute an reiner CAC betrug 90%.

### Beispiel 2

In die vorbereitete Apparatur wurden 16,6 g DBA (98 - 99%; 50 mmol DBA 100%) eingewogen und in 115 ml entionisiertem Wasser, aufgeschlämmt, und der Kolbeninhalt wurde im Oelbad auf 45°C erwärmt und dann 10,0 ml Kalilauge (48 Gew.%, 127 mmol) zugefügt. Nach 10 minütigem Rühren wurde die DBA klar gelöst. Der pH-Wert der Lösung betrug 12,4. Mit 0,5 ml konzentrierter Salzsäure (6 mmol) wurde auf pH 9,9 neutralisiert.

In einen 250 ml-Tropftrichter wurden 29,7 g Triphosgen (100 mmol) eingewogen und in 76 ml absolutem Toluen gelöst. Diese Lösung wurde unter gutem Rühren innert einer Stunde 45 Minuten bei 45°C zur Lösung des DBA-Dikaliumsalzes bei pH 9,5 bis 10,5 zugetropft. Man erhielt eine braune Emulsion. Neutralisiert wurde mit 92 ml 48 Gew.%iger Kalilauge (1,168 mol KOH). Der pH-Wert betrug 9,9. Da Triphosgen, gelöst in Toluen, mit wässrigen Lösungen weniger schnell abreagiert, wurde noch 30 Minuten nachgerührt. Dabei fiel der pH-Wert auf 9,7. Mit 2,0 ml 48 Gew.%iger Kalilauge (25,4 mmol KOH) wurde auf pH 9,9 neutralisiert. Der pH-Wert blieb konstant bei pH 9,94 stehen. Dies bedeutete, dass das gesamte Triphosgen abreagiert hatte. Der totale Zeitbedarf für die Umsetzung von DBA zu CAC betrug etwa 2,5 Stunden.

Danach wurde der Kolbeninhalt möglichst vollständig in einem 1000 ml-Scheidetrichter überführt. Man erhielt sehr schnell eine gute Phasentrennung. In der oberen, dunkelbraun gefärbten Toluenphase befanden sich die Verunreinigungen. Die Menge dieser Phase betrug 62,6 g. Die untere, schwach gelb gefärbte Phase enthielt die alkalische wässrige Reaktionslösung. Das Volumen betrug etwa 300 ml. Diese Lösung wurde in einen 500 ml-Tropftrichter überführt.

Zur Aufarbeitung (direkte Fällung) wurden in einem mit einem mechanischen Rührer, Thermometer und pH-Elektrode versehenen 750 ml-Vierhalssulfierkolben, 45 ml konzentrierte Salzsäure (36 Gew.%; 522 mmol) vorgelegt, etwa 20 mg rohe CAC zum Animpfen zugefügt und dann im Oelbad auf etwa 40°C erwärmt. Danach wurde mittels Tropftrichter die alkalische wässrige Reaktionslösung unter kräftigem Rühren zur konzentrierten Salzsäure zugetropft. Unter CO₂₋Entwicklung bildeten sich schon nach Zugabe von etwa 20 ml dieser Reaktionslösung CAC-Kristalle. Die Zutropfgeschwindigkeit wurde auf 5 ml/Min. eingestellt. Nach 30 Minuten ist alles zugetropft worden.

Rohe CAC bildete einen feinkristallinen Niederschlag. Man liess nach 15 Minuten bei 40°C rühren, danach abkühlen und weitere 30 Minuten im Eisbad bei etwa +5°C rühren. Der pH-Wert der wässrigen Lösung betrug 0,64.

Danach wurde der Ansatz durch eine P4-Glassinterfritte filtriert, der gelbbraune, kristalline Rückstand dreimal mit je 40 ml entionisiertem Wasser neutral gewaschen und im Trockenschrank unter Hochvakuum bei 80°C bis zur Gewichtskonstanz getrocknet.

| Ergebnisse | |
|---|---|
| rohe CAC: | 16,7 g = 94,35% der Theorie |
| Gehalt an reiner CAC: | 94,1% (nach HPLC) |
| Ausbeute an reiner CAC: | 88% der Theorie |

Die Menge der Toluen-Phase betrug 62,6 g. Nach Einengen unter vermindertem Druck verblieb etwa 0,7 g an braunen, undefinierbaren Nebenprodukten. Dies entsprach 4,2% bezogen auf eingesetzte DBA. Erhalten wurden 60 g Toluen-Destillat; dies entsprach etwa 69 ml Toluen bzw. 90% der eingesetzten Lösungsmittelmenge an Toluen (76 ml).

Im wässrigen Filtrat befanden sich 1,193 mol Kaliumchlorid. Dies entsprach einer Salzmenge von 88,94 g Kaliumchlorid. Ausserdem enthielt dieses Filtrat nach DC noch geringe Mengen an CAC (max. 0,5 - 1%), DBA (max. 1%) sowie nicht näher untersuchte Nebenprodukte (etwa 2%). Das wässrige Filtrat wurde verworfen.

## Patentansprüche

1. Verfahren zur Herstellung von 2-Oxo-1,3-dibenzyl-cis-4,5-imidazolidindicarbonsäure ausgehend von meso-2,3-Bis(benzylamino)-bernsteinsäure-Dialkalimetallsalz, dadurch gekennzeichnet, dass man das meso-2,3-Bis(benzylamino)-bernsteinsäure-Dialkalimetallsalz mit Triphosgen in einem aus wässriger Alkalilauge und einem organischen Lösungsmittel bestehenden Zweiphasen-Lösungsmittelsystem bei einer etwa 50°C nicht übersteigenden Temperatur umsetzt und das daraus resultierende, in der wässrigen Phase befindliche 2-Oxo-1,3-dibenzyl-cis-4,5-imidazolidindicarbonsäure-Dialkalimetallsalz durch Ansäuern in die gewünschte 2-Oxo-1,3-dibenzyl-cis-4,5-imidazolidindicarbonsäure umwandelt.

2. Verfahren nach Anspruch 1, dadurch gekennzeichnet, dass als Ausgangsmaterial das meso-2,3-Bis(benzylamino)-bernsteinsäure-Dinatriumsalz oder -Dikaliumsalz, vorzugsweise das letztere, verwendet wird.

3. Verfahren nach Anspruch 1 oder 2, dadurch gekennzeichnet, dass zur Zubereitung des meso-2,3-Bis(benzylamino)-bernsteinsäure-Dialkalimetallsalzes die Säure selber in Wasser aufgeschlämmt und die resultierende Aufschlämmung mit Alkalimetalllauge, vorzugsweise Kalilauge, versetzt wird, um eine klare, alkalische wässrige Lösung des erwünschten Dialkalimetallsalzes zu erhalten.

4. Verfahren nach Anspruch 3, dadurch gekennzeichnet, dass die Konzentration des gebildeten meso-2,3-Bis(benzylamino)-bernsteinsäure-Dialkalimetallsalzes in der klaren, alkalischen wässrigen Lösung etwa 5 bis etwa 20 Gewichtsprozent, vorzugsweise etwa 10 bis etwa 15 Gewichtsprozent, bezogen auf das Gesamtgewicht der Lösung bei pH etwa 9 bis etwa 14 beträgt.

5. Verfahren nach einem der Ansprüche 1 bis 4, dadurch gekennzeichnet, dass als organisches Lösungsmittel ein aprotisches organisches Lösungsmittel, insbesondere ein aliphatischer oder cyclischer Ether, z.B. Diethylether bzw. Tetrahydrofuran oder Dioxan; ein aliphatischer oder alicyclischer Kohlenwasserstoff, z.B. Hexan, Octan bzw. Cyclohexan; ein aliphatischer oder cyclischer Ester, z.B. Ethylacetat bzw.γ-Butyrolacton; oder ein aromatischer Kohlenwasserstoff, z.B. Benzen oder Toluen, vorzugsweise Tetrahydrofuran oder Toluen, verwendet wird.

6. Verfahren nach einem der Ansprüche 1 bis 5, dadurch gekennzeichnet, dass zur Bildung des Zweiphasen-Lösungsmittelsystems, im welchem das meso-2,3-Bis(benzylamino)-bernsteinsäure-Dialkalimetallsalz und das Triphosgen miteinander reagieren, eine gemäss Anspruch 3 zubereitete klare alkalische wässrige Lösung des meso-2,3-Bis(benzylamino)-bernsteinsäure-Dialkalimetallsalzes mit einer Lösung des Triphosgens im organischen Lösungsmittel zusammengebracht wird.

7. Verfahren nach Anspruch 6, dadurch gekennzeichnet, dass die klare alkalische wässrige Lösung und/oder die Lösung des Triphosgens auf eine erhöhte Temperatur im Bereich von etwa 30 bis etwa 50°C, vorzugsweise im Temperaturbereich von etwa 40 bis etwa 45°C, erwärmt worden ist.

8. Verfahren nach einem der Ansprüche 1 bis 7, dadurch gekennzeichnet, dass das Molverhältnis Triphosgen : meso-2,3-Bis(benzylamino) -bernsteinsäure-Dialkalimetallsalz etwa 0,33 : 1 bis etwa 10 : 1, vorzugsweise etwa 1,5 : 1 bis etwa 5 : 1, besonders bevorgzugt etwa 2 : 1 bis etwa 4 : 1, beträgt.

9. Verfahren nach einem der Ansprüche 1 bis 8, dadurch gekennzeichnet, dass während der Umsetzung im Zweiphasen-Lösungsmittelsystem der pH-Wert der wässrigen Phase im Bereich von etwa 8,5 bis etwa 13, vorzugsweise im Bereich von etwa 9,5 bis etwa 10,5, gehalten wird.

10. Verfahren nach einem der Ansprüche 1 bis 9, dadurch gekennzeichnet, dass die Umsetzung bei einer Temperatur im Bereich von etwa 30 bis etwa 50°C, vorzugsweise im Temperaturenbereich von etwa 40 bis etwa 45°C, erfolgt.

11. Verfahren nach einem der Ansprüche 1 bis 10, dadurch gekennzeichnet, dass zum Ansäuern des erhaltenen, in der wässrigen Phase befindlichen 2-Oxo-1,3-dibenzyl-cis-4,5-imidazolidindicarbonsäure- Dialkalimetallsalzes eine Mineralsäure, insbesondere Salzsäure, Bromwasserstoffsäure oder Schwefelsäure, vorzugsweise Salzsäure, verwendet wird.
